# EUROPEAN PATENT APPLICATION

(11) **EP 3 848 708 A1**
(43) Date of publication of application: **14.07.2021**
(21) Application number: 20151438.7
(22) Date of filing: 13.01.2020
(51) Int. Cl.: G01N 33/52, C12Q 1/37, C12Q 1/44, G01N 33/569

(54) **REAGENT FORMULATION FOR LEUKOCYTE TEST STRIP**

(71) Applicant: Roche Diagnostics GmbH, 68305 Mannheim (DE); F. Hoffmann-La Roche AG, 4070 Basel (CH)
(72) Inventor: HORN, Carina, 68305 Mannheim (DE); REGN, Michael, Andreas, 68305 Mannheim (DE)
(74) Representative: Immendörfer-Rühle, Ulrike

(57) **Abstract**

The invention relates to a composition for determining the presence of an esterolytically and/or proteolytically active analyte in an isolated body fluid sample, in particular leukocytes, comprising a diazonium salt, an indoxyl ester capable of producing a colorimetrically detectable reaction product with said diazonium salt, taurine and divalent anions. It further relates to a test device containing said composition, to a method for detecting an esterolytically and/or proteolytically active analyte and to the use of said composition for the detection of an esterolytically and/or proteolytically active analyte in a body fluid sample.

## Description

The present invention concerns a composition, a test device, its use and a method for determining the presence of an esterolytically and/or proteolytically active substance(s) or analyte(s) present in a body fluid.

### Background of the Invention

The detection of analytes in body fluids, such as urine, using test strip-based devices has been known for several decades, for review see e.g. Kompendium der Urinanalyse 2014, Roche Diagnostics (in German), https://www.roche.de/res/content/7807/urinanalvse-kompendium.pdf or http://www.cobas.roche.it/content/dam/cobas_com/pdf/product/urinalysis-compendium.pdf (in English). Test strip based devices usually contain a plurality of test fields, each dedicated for detection of a specific analyte or parameter. Typical analytes or parameters are specific gravity, pH, leukocytes, nitrate, protein, glucose, ketones, urobilinogen, bilirubin, blood (*e.g.* erythrocytes) and albumin. An analyte of specific interest is the class of leukocytes and the enzymes associated with or contained in leukocytes. In *in vitro* diagnostics, the presence of leukocytes in urine (leukocyturia) provides an important hint towards inflammatory diseases of the efferent urinary tract and kidneys, such as bacterial and abacterial infections and parasite infestations. Abacterial leukocyturia can also constitute important evidence for the presence of tuberculosis or tumors.

Leukocyte test strips or test fields known in the art are usually based on the detection of the esterase and/or protease activity of a subclass of leukocytes, *i.e.* mainly granulocytes, that are excreted in the urine. The test field (also called test zone) of such a strip device contains an indoxyl ester, which is cleaved by the granulocyte esterase. The free indoxyl reacts with a diazonium salt to form a colored dye which can be visually detected - either by patient's or operator's eyes or by using an optical detection instrument. The indoxyl ester present in such a leukocyte test field needs both a buffered environment so that the esterase maintains its enzymatic activity and can cleave the substrate and also needs a stabilizing compound preventing the ester from premature degradation in absence of the analyte thus assuring a long shelf life. In the prior art, leukocyte test field material therefore contains boric acid or borate (salt of boric acid), which both stabilizes and buffers the indoxyl ester, see *e.g.* US 5,015,572. However, boric acid and its salts are critical from an environmental perspective. In view of the increasing demands regarding ecological rules and regulations such as the European Chemicals Regulation REACH (Registration, Evaluation, Authorisation and Restriction of Chemicals) omission, reduction or replacement of such critical substances is required.

It is against the above background that the embodiments of the present disclosure provide certain unobvious advantages and advancements over the prior art.

In particular, an alternative reagent composition and test device for the detection of esterolytically and/or proteolytically active substances or analytes present in body fluids such as urine, especially an environmentally acceptable alternative to the use of boric acid and its salts for such *in vitro* diagnostics purposes, are herein introduced.

### Summary of the Invention

A composition for determining the presence of an esterolytically and/or proteolytically active analyte in an isolated body fluid sample, comprising a diazonium salt, an indoxyl ester capable of producing a colorimetrically detectable reaction product with said diazonium salt, taurine and divalent anions is introduced. A a test device containing said composition, a method for detecting an esterolytically and/or proteolytically active analyte and the use of said composition for the detection of an esterolytically and/or proteolytically active analyte in a body fluid sample are also introduced.

### Description of the Figures

**Figure 1**: Principle of color reaction on a test strip, illustrated by chemical compounds: The leukocytes excreted in the urine are almost exclusively granulocytes, the esterase activity of which is detected in the test strip reaction. The test zone contains an indoxyl ester, which is cleaved by the granulocyte esterase. The free indoxyl reacts with a diazonium salt to form a violet dye.
**Figure 2**: Initial buffer screening for alternatives to boric acid, examples PIPES and H₂PO₄/HPO₄ (Good's buffers).
**Figure 3**: Comparison of remission % in samples spiked with leukocyte esterase of boric acid, taurine and taurine+ magnesium sulfate. The X axis shows the esterase concentration as units per deciliter (U/dl).
**Figure 4**: Graphical illustration of table 2, storage for 26 weeks at 4°C. The X axis shows the esterase concentration as units per deciliter (U/dl).
**Figure 5**: Graphical illustration of table 3, heat stress for 26 weeks at 35°C. The X axis shows the esterase concentration as units per deciliter (U/dl).
**Figure 6**: Functional curve of urine spiked with esterase, comparison of boric acid with taurine/magnesium sulfate. The X axis shows the esterase concentration as units per deciliter (U/dl).
**Figure 7**: Native urine from pathological clinical samples is applied. This figure shows the correlation between the prior art composition (% remission of composition with boric acid) and the new inventive composition (% remission of composition with taurine and MgSO₄).

### Detailed Disclosure of the Invention

As described in the background section, in view of increasing environmental and regulatory demands producers such as chemical, pharmaceutical and diagnostic companies are required to replace substances in their production processes and their products, that are not easily degradable or otherwise toxic, by less critical compounds. For the production of strip-based *in vitro* diagnostic tests, boric acid and its salts qualify as substances to be avoided in future production. Boric acid/borate serves as a buffer in the weak alkaline range (around pH 8) and as a stabilizer for the indoxyl compound that is needed to produce an optically detectable color signal on a test strip.

Extensive tests with ecologically tolerable buffer systems for replacement of boric acid/borate known to a person skilled in the art such as phosphate, PIPES or HEPES failed to provide satisfying results. Either the buffering function was insufficient or the indoxyl ester led to a change in color or discoloration right after preparation of the test strip material. Taurine has been described as an alternative to borates (see *e.g.* chapter 5.2 in "Buffers for pH and metal ion control", Chapman and Hall laboratory manuals in physical chemistry and biochemistry, D. Perrin Springer Science & Business Media, 2012). However, taurine alone does not satisfy the need of stabilization of a composition used for impregnating test strip material. Surprisingly, when a combination of taurine and divalent anions as buffer and stabilizer was added to the indoxyl ester-containing impregnating solution for the test strip material the stability and performance of the impregnated test strip material was comparable to the traditional way that applies borate to achieve these effects. Importantly, also the detection/reference ranges for "Normal": < 10 leukocytes/µL, "Borderline": 10-20 leukocytes/µL and "Pathological": > 20 leukocytes/µL could be reached with the new composition that ensures an excellent sensitivity and specificity of the leukocyte test. Thus, the obtained detection/reference ranges are fully comparable to those obtained with the existing test composition.

The invention therefore concerns a composition for determining the presence of an esterolytically and/or proteolytically active analyte in an isolated body fluid sample, comprising a diazonium salt, an indoxyl ester capable of producing a colorimetrically detectable reaction product with said diazonium salt, taurine and divalent anions.

The term composition means that several chemical substances or compounds are present together in a mixture. The composition may be a dry composition, *i.e*. in the absence of a solvent such as water. The composition may also be a liquid composition, *i.e.* all compounds are dissolved in a solvent such as water or a liquid buffer.

An esterolytically and/or proteolytically active analyte is a substance, usually dissolved in a liquid such as a body fluid, that is able to cleave ester bonds and/or amide bonds. In an embodiment, the analyte is a leukocyte. Leukocytes in urine, in particular granulocytes, a subclass of leukocytes, possess several esterolytically and proteolytically active enzymes that are able to cleave ester compounds such as indoxyl ester into the corresponding alcohol and organic acid or cleave the target chemical amide bond into an amino compound and an organic acid. Both types of chemical covalent bonds are known to a person skilled in the art. Further examples of proteolytic enzymes that may be present in an isolated biological sample are elastase, chymotrypsin or trypsin. When the esterolytically and/or proteolytically active analyte reacts with an indole ester, the resulting hydroxyl compound (the alcohol) reacts with the diazonium salt also present in the composition, resulting in a colorimetrically detectable azo dye.

An isolated body fluid sample is a term well-known in the art. In an embodiment, an isolated body fluid sample is urine, blood plasma, blood serum or liquor. In principle all biological samples that do not show significant absorption in the range of the photometrically detectable signal can be used.

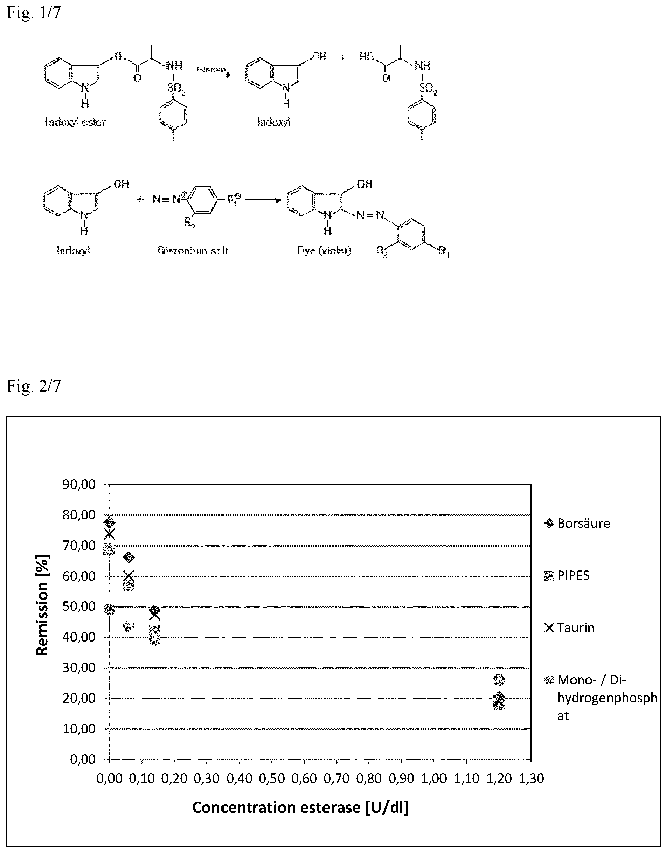

As indoxyl ester which forms one part of the colored detectable end product, any indoxyl ester may be used that is able to react with an ester or amide bound cleaving enzyme, such as an esterase like leukocyte esterase or a protease, so that a hydroxy compound (free OH group) and a carboxylic compound is built. In the case of a protease, a free OH group and an amide is produced. The free OH group then reacts with a diazonium part of a diazonium salt in such a way that a colorimetrically detectable end product is obtained. In an embodiment, said indoxyl ester comprises 3-(N-toluene-4'-sulphonyl-L-alanyloxy)-indole. In another embodiment, said indoxyl ester is 3-(N-toluene-4'-sulphonyl-L-alanyloxy)-indole. For illustration see Fig. 1.

As diazonium salt, any diazonium salt may be used that is able to react with a free OH group, e.g. resulting from enzymatic cleavage of an an indoxyl ester, in such a way that a colorimetrically detectable end product is obtained. In an embodiment, said diazonium salt comprises 2-methoxy-4(N-morpholino)-benzene-diazonium tetrachlorozincate. In a further embodiment, said diazonium salt is 2-methoxy-4(N-morpholino)-benzene-diazonium tetrachlorozincate.

A further ingredient of the composition according to the invention is taurine, the chemical name of which is 2-aminoethanesulfonic acid.

Yet another ingredient of the composition are divalent anions, e.g. sulfate (SO₄²⁻), sulfite (SO₃²⁻), carbonate (CO₃²⁻) or oxide (O²⁻) ions. In an embodiment the divalent anions are sulfate anions. As counter ions for the divalent anions any metal cation can be chosen such as *e.g.* Mg²⁺, Ca²⁺, Ni²⁺ or Mn²⁺. In an embodiment, the metal cation is Mg²⁺. In an embodiment, the sulfate ions stem from magnesium sulfate or calcium sulfate, in an embodiment from magnesium sulfate.

In addition to the ingredients of the composition, further additives can be present. Examples of these are additional buffer substances, wetting agents, stabilizers and/or activators.

With regard to the applied concentrations of the ingredients, the molar ratio of taurine and sulfate ions may range between 1:1, in the meaning of equal molarities, *i.e.* equimolar and a three-fold access (3:1) of taurine over sulfate ions. The molar excess of both taurine and sulfate ions over the indoxyl ester may range between 60 to 90-fold, in an embodiment between 65 to 85-fold, in an embodiment 70-fold, all ranges with a tolerance of +/- 10%.

In view of the environmental concern it is beneficial if the composition according to the invention does not contain boric acid or borate ions. This means that the composition is free from boric acid or borate ions, *i.e.* that their presence cannot be detected by standard analytical methods known in the art like *e.g.* GC-MS (gas chromatography mass spectrometry).

In an embodiment, the ingredients of the composition are stored in immobilized form on a test device. In a further embodiment, the ingredients of the composition are stored in dry form on a test device.

In a further embodiment, the ingredients of the composition are stored in one reagent-containing layer on the test device. This means that all components of the composition described further above, *i.e.* an indoxyl ester, a diazonium salt, taurine and divalent anions, are present in the same reagent-containing layer or compartment. The joint presence of all components of the composition in one common compartment is advantageous as the product resulting from reaction of the analyte with the indoxyl ester can quickly react further with the diazonium salt present in the same compartment to produce a colored end product. The reagents which participate in the reaction are mixed with one another after the reagent-containing layer is contacted with the liquid sample to be investigated, so that the reagents go at least partly in solution.

In yet another embodiment, the test device comprises at least two reagent-containing layers that are stacked on top of each other. In this embodiment the diazonium salt is stored in a first of the at least two reagent-containing layers and the indoxyl ester together with both said taurine and said sulfate ions are stored in a second of the reagent-containing layers. In an embodiment, the first reagent-containing layer is located or placed on top of the second reagent-containing layer. In an embodiment, the diazonium salt is at the top of the test device and the mixture of indoxyl ester, taurine and divalent anions, e.g. sulfate ions, is at the bottom. The different reagent layers are arranged in such a way that liquid exchange between them is possible in order to enable the complete chemical reaction when the test device is wetted with the sample. The test sample wets the indoxyl ester together with both said taurine and said sulfate ions, bringing all components at least partly into solution within the layer, where sulfate ions and taurine provide a stabilized and buffered environment for the indoxyl ester. When an analyte, e.g. leukocyte esterase, is present in the test sample, it reacts with the indoxyl ester, so that a hydroxyl compound results as a cleavage product. Subsequently or in parallel, the test sample wets the diazonium salt-containing layer so that the color reaction between the hydroxyl group of the cleaved indoxyl ester and the diazonium salt can take place. As a result, a colored product is received. Usually, to facilitate handling of the test device, a synthetic material carrier such as a stiff plastic film or plastic foil is used as a bottom part to support all reagent-containing layers. The fixing of the stack of reagent layers onto such a support layer is known in prior art and *e.g.* described in US 5,015,572.

In an embodiment, the reagent containing layers are additionally covered by a liquid permeable fixation layer such as a nylon mesh.

As base materials for creating different reagent-containing layers any material which is conventionally used for this purpose can be applied, in an embodiment absorbent or swellable material, in another embodiment porous material or non-porous material, cellulose, filter paper, synthetic fibers, glass fibers, polyvinyl esters, polyamide films or xanthan gum films.

In an embodiment, during preparation of the test device in a two-step approach, the pH of the soaking or impregnating solution containing taurine and sulfate (which are added in the first step) ranges in the slightly alkaline region, *i.e*. around pH 8, in an embodiment between 8.2 and 8.4. In a second step, the indoxylester having a pH around 6 is added to the impregnating solution so that the final pH during measurement, *i.e.* in the sample, typically ranges below pH 8, in an embodiment between 7.5 and 7.9. Thus, the combination of taurine and sulfate is helpful in buffering and stabilizing the composition during preparation of the test device as well as during the measurement of a sample.

In an embodiment, a test device is a carrier-bound detection system with at least one reagent-containing layer which is positioned on a carrier to permit a liquid flow into or through this layer. Carrier-bound detection systems, e.g. in the form of a test strip, have proven to be highly suitable for quick and reliable detection of infections and disturbances of the urogenital tract as such a test strip only has to be dipped into the urine sample for about one second. The test strip is then removed from the sample and the final color develops within about 1 to 2 minutes. Usually, the developed color is compared with a comparison color chart and the approximate number of leukocytes per ml of sample can be determined on the basis of color formation. Detection of the received color may be carried out by reading off the color visually or by measuring the remission (which is the diffuse reflection of light) photometrically as known by a person skilled in the art.

In an embodiment, the at least one layer containing a plurality of reagents which effect a colorimetrically detectable signal in the presence of an esterolytically and/or proteolytically active analyte comprises a composition according to the embodiments described further above. The test device may also comprise a plurality of layers, *i.e.* comprising at least a) a first layer containing as reagent a diazonium salt, in an embodiment 2-methoxy-4(N-morpholino)-benzene-diazonium tetrachlorozincate, and b) a second layer containing as reagent an esterolytically or proteolytically cleavable indoxyl ester, taurine and sulfate ions, in an embodiment 3-(N-toluene-4'-sulphonyl-L-alanyloxy)-indole, taurine and sulfate ions.

In yet another embodiment, the invention concerns a method for detecting the presence of an esterolytically and/or proteolytically active analyte in an isolated body fluid sample comprising contacting an isolated body fluid sample with a test device as disclosed further above, and determining a colorimetric signal as an indication of the presence of said analyte. The definitions and explanations for the setup of the test device, the sample and the composition as provided further above also apply for the method according to the invention.

A further embodiment is the use of the composition as described within this specification for the detection of leukocytes in an isolated body fluid sample.

Yet another embodiment is a method of producing a composition for determining the presence of an esterolytically and/or proteolytically active analyte in an isolated body fluid sample, comprising a diazonium salt, an indoxyl ester capable of producing a colorimetrically detectable reaction product with said diazonium salt, taurine and divalent anions. In an embodiment, the method of producing said composition is a two-step process, wherein in a first step divalent anions *e.g.* in the form of magnesium sulfate, and taurine are mixed in aquous solution, followed by an impregnating step of a paper fleece which is then dried. As a second step, said indoxyl ester is dissolved by a solvent comprising water and alcohol. The impregnated paper of step one is then impregnated with the indoxyl ester solution, followed by drying of said paper.

The following examples, figures and sequences are provided to aid the understanding of the present invention, the true scope of which is set forth in the appended claims. It is understood that modifications can be made in the procedures set forth without departing from the scope of the invention.

Although the foregoing invention has been described in some detail by way of illustration and example for purposes of clarity of understanding, the descriptions and examples should not be construed as limiting the scope of the invention.

### Examples

### Example 1: Preparation of composition and test device

The composition used for producing a leukocyte test device was prepared in a two-step process. To 44 kg destilled water the following components (stirring and dissolving each component one after another) were individually added: 1.3 kg taurine, 0.2 kg sodium hydroxide, 2.5 kg MgSO₄^{∗}7H₂O and 3.465 kg hydrochloric acid. The pH of this solution was between 8.2 and 8.4. This mixture was used for impregnating paper fleece 23 SL III (about 350g/m², Schleicher & Schüll, Germany) which was then dried.

In the second step, to 61,6 kg of ethanol were added in a step-wise process including stirring: 72.2 g TSA-indole (3-(N-toluene-4'-sulphonyl-L-alanyloxy)-indole), 7.6 kg phosphoric acid trimorpholide, 2.4 kg destilled water, 1.7 kg decanol, 10.3 kg methanol and 38 g diazonium-chloro-zincate. The pH of this solution was around pH 6. The dried paper fleece of step 1 was impregnated with the solution of step 2 and then finally dried again. The final pH within the test field composition during measurement, *i.e.* when the sample was added, was below pH 8, ranging from 7.5 to 7.9. This composition is able to detect leukocytes in isolated body fluids.

Table 1 shows the final concentrations of taurine, magnesium sulfate and the TSA-indole in the soaking solution and in the final dried paper:

**Table 1:**

| Compound | Concentration in dry paper g/m² | Concentration in soaking solution mmol/L | Ratio |
|---|---|---|---|
| Taurine | 8.05 | 200 | 80 |
| MgSO₄^{∗}7H₂O | 15.90 | 200 | 80 |
| TSA-indole | 0.21 | 2.5 | 1 |

The buffering of the composition during preparation was maintained by the addition of taurine. For stabilization of the diazonium salt, MgSO₄ was added. Alternative buffering systems such as PIPES or phosphate did not show any considerable effect.

Even the freshly prepared composition showed a strong decrease in remission at low analyte concentrations, see Fig. 2, for various Good's buffers, in comparison with boric acid. Further buffer systems (HEPES) already showed strong decoloration directly after soaking the paper with the composition.

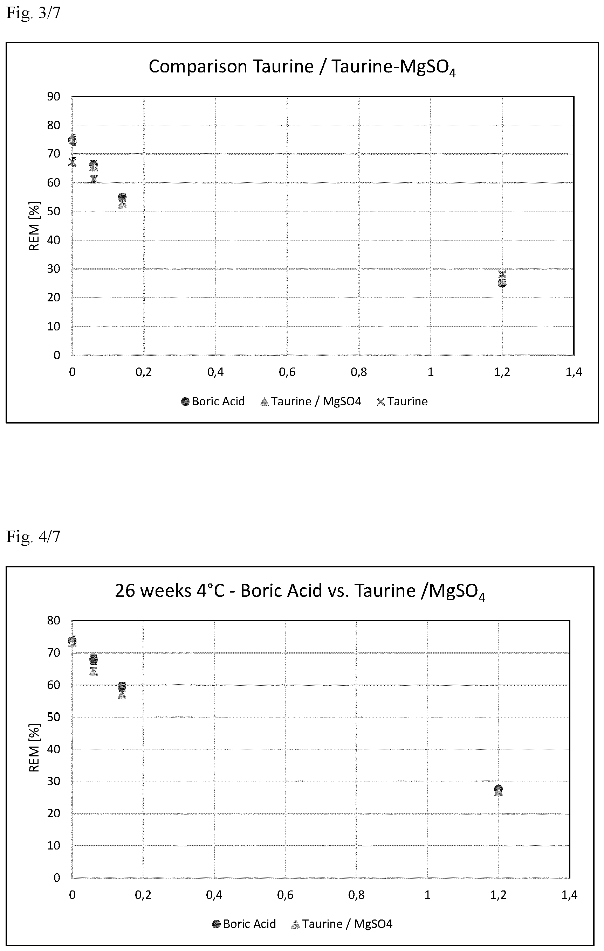

As can be seen from Fig. 3, without sulfate anions a decrease of remission can be observed at low leukocyte counts which can be explained by ester cleavage in the absence of any enzyme. The color of the resulting indoxyl is quite similar to the reaction color and is therefore also detected as reaction color. This phenomenon might falsely be interpreted as a high concentration of leukocytes, see in particular leukocyte esterase concentrations of 0 U/dl and 0.06 U/dl.

In addition, also the pH of the composition needs to be in the range of pH 8.2 to pH 8.4. This composition shows an excellent stability against heat stress (26 weeks at 35°C). However, when the pH exceeds pH 8.4 the composition shows inferior stability which is comparable to the situation when no MgSO₄ is added.

Tables 2 and 3 show that the storage stability of the composition according to the invention is well in line with the stability of the composition of prior art that contains boric acid.

**Table 2: Remission % after 26 weeks storage at 4°C (control)**

| Esterase activity [U/dl] | 0 U/dl | 0.06 U/dl | 0.14U/dl | 1.20U/dl |
|---|---|---|---|---|
| Original (Boric acid) | 73.7 | 67.9 | 59.5 | 27.6 |
| Taurine + MgSO₄ | 73.2 | 64.3 | 56.9 | 26.9 |

**Table 3: Remission % after 26 weeks storage under heat stress at 35°C**

| Esterase activity [U/dl] | 0 U/dl | 0.06 U/dl | 0.14U/dl | 1.20U/dl |
|---|---|---|---|---|
| Original (Boric acid) | 72.8 | 64.9 | 56.6 | 25.9 |
| Taurine + MgSO₄ | 71.1 | 62.9 | 54.8 | 29 |

For the data shown in Tables 2 and 3, esterase activity corresponds to activity of lysed leukocytes from Leukocyte Esterase, Unsonicated commercially available from LEE BIOSOLUTIONS.

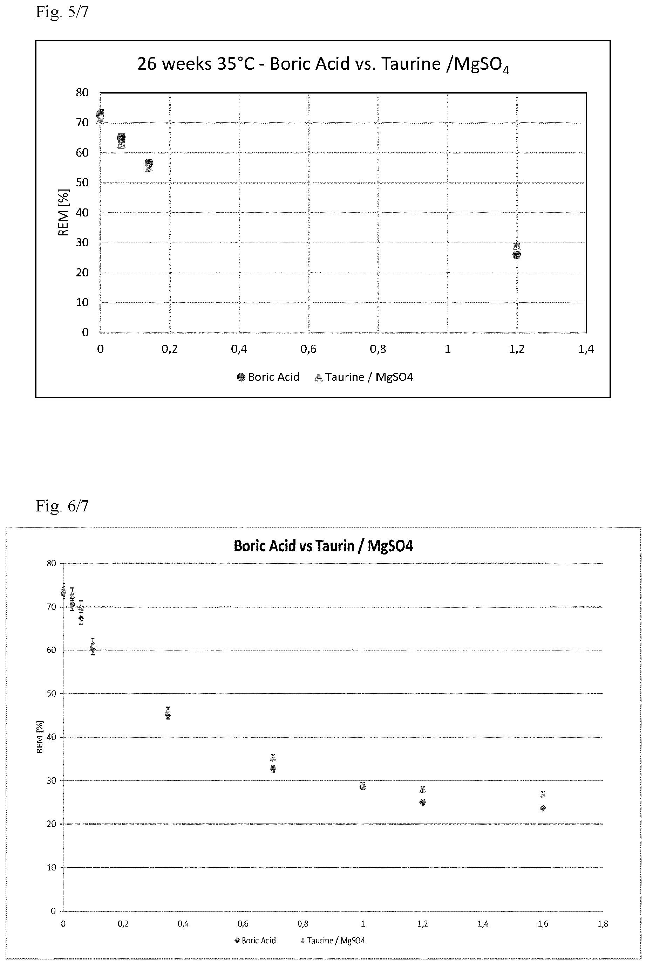

Figures 4 and 5 show the respective graphical illustration of tables 2 and 3.

### Example 2: Analyte detection using composition in comparison with prior art

A test strip using the composition prepared according to example 1 and the test device set-up as also described in example 1 (invention) was produced and measured in a cobas u411 test device (Roche Diagnostics GmbH, Germany), using the Combur 10M test strip (Roche Diagnostics GmbH, Germany) as reference composition with boric acid. Normal urine was spiked with esterase to imitate the presence of leukocytes. Figs. 4 and 5 show that both compositions, *i.e.* the one with boric acid and the one of the invention without boric acid but with taurine and sulfate, show a very similar behavior with regard to remission %. In other words, the combination of divalent anions, in particular of sulfate anions, with taurine provides an environmentally fully acceptable alternative to the traditional boric acid for stabilizing and buffering a composition meant to detect the presence of an esterolytically and/or proteolytically active analyte such as a leukocyte.

Fig. 6 shows a functional curve of urine spiked with esterase, comparison of boric acid vs. taurine/MgSO₄ according to the invention. The X axis shows the esterase concentration in units per dezi litre (U/dl). It can be seen that the range of remission % is very similar to each other, again supporting the composition of the invention as an ecologically suitable alternative to the use of boric acid. The performance of the composition is not sacrificed by using the environment-friendly combination of taurine and magnesium sulfate.

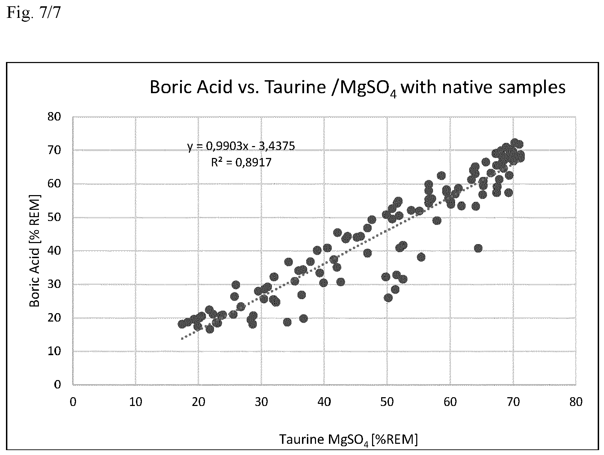

In addition to the spiked samples also native urine (pathological clinical samples) was tested. Also here the correlation between the old and the new composition was very good. It was found that the pH window between pH 8.2 and 8.4 is the optimum for the composition. The results for this correlation is illustrated by Fig. 7.

## Claims

1. Composition for determining the presence of an esterolytically and/or proteolytically active analyte in an isolated body fluid sample, comprising a diazonium salt, an indoxyl ester capable of producing a colorimetrically detectable reaction product with said diazonium salt, taurine and divalent anions.

2. Composition as claimed in claim 1, wherein the divalent anions are sulfate anions.

3. Composition as claimed in any of the preceding claims, wherein said diazonium salt comprises 2-methoxy-4(N-morpholino)-benzene-diazonium tetrachlorozincate and/or wherein said indoxyl ester comprises 3-(N-toluene-4'-sulphonyl-L-alanyloxy)-indole.

4. Composition as claimed in any of the preceding claims, wherein the esterolytically or proteolytically active analyte is a leukocyte.

5. Composition as claimed in any of the preceding claims, wherein the ingredients of said composition are stored in immobilized form on a test device.

6. Composition as claimed in claim 5, wherein the ingredients of said composition are stored in one reagent-containing layer on said test device.

7. Composition as claimed in claim 5, wherein said test device comprises at least two reagent-containing layers that are stacked on top of each other and wherein said diazonium salt is stored in a first of said reagent-containing layers and said indoxyl ester together with both said taurine and said sulfate ions are stored in a second of said reagent-containing layers.

8. Composition as claimed in claim 7, wherein said first reagent-containing layer is located on top of said second reagent-containing layer.

9. Composition as claimed in any of claims 6 to 8, wherein said reagent-containing layers are covered by a liquid permeable fixation layer.

10. Composition as claimed in any of the preceding claims, wherein said sulfate ions are derived from magnesium sulfate or calcium sulfate.

11. Composition as claimed in any of the preceding claims, wherein said composition is free from boric acid or borate ions.

12. Test device comprising a reagent-containing layer positioned on a carrier to permit a liquid flow into said layer, said layer containing a plurality of reagents which effect a colorimetrically detectable signal in the presence of an esterolytically and/or proteolytically active analyte, wherein said at least one layer comprises a composition as claimed in any of claims 1 to 11.

13. Test device comprising a plurality of reagent-containing layers positioned on a carrier to permit a liquid exchange between said layers, and a composition as claimed in any of claims 1 to 11.

14. Method for detecting the presence of an esterolytically and/or proteolytically active analyte in an isolated body fluid sample comprising contacting an isolated body fluid sample with a test device according to any of claims 12 or 13, and determining a colorimetric signal as an indication of the presence of said analyte.

15. Use of the composition according to any of claims 1 to 11 for the detection of leukocytes in an isolated body fluid sample.
